# EUROPEAN PATENT SPECIFICATION

(11) **EP 2 095 126 B1**
(45) Date of publication and mention of the grant of the patent: **17.03.2010**
(21) Application number: 06821550.8
(22) Date of filing: 15.12.2006
(51) Int. Cl.: G01N 33/569, G01N 33/68

(54) **A METHOD FOR DETECTING HUMAN PARVOVIRUS ANTIGEN**
VERFAHREN ZUM NACHWEIS VON MENSCHLICHEM PARVOVIRUS-ANTIGEN
PROCÉDÉ DE DÉTECTION D'ANTIGÈNE PARVOVIRUS HUMAIN

(43) Date of publication of application: 02.09.2009
(73) Proprietor: Biotrin Intellectual Properties Limited, Mount Merrion County Dublin (IE)
(72) Inventor: BUTLER, Mary Bernadette, Saggart, County Dublin (IE); CORCORAN, Amanda Marie, Ashbourne, County Meath (IE); ELLIOT, Ian Gordon, Celbridge, County Kildare (IE); KERR, Shane, Leopardstown, County Dublin (IE); KILTY, Cormac Gerard, Sandycove, County Dublin (IE)
(74) Representative: Ryan, Anne Mary
(86) International application number: PCT/IE2006/000141
(87) International publication number: WO 2008/072216

(56) References cited:
- O'NEILL H J ET AL: "TWO ANTI-PARVOVIRUS B19 IGM CAPTURE ASSAYS INCORPORATING A MOUSE MONOCLONAL ANTIBODY SPECIFIC FOR B19 VIRAL CAPSID PROTEINS VP1 AND VP2" ARCHIVES OF VIROLOGY, vol. 123, no. 1-2, 1992, pages 125-134, XP009082200 ISSN: 0304-8608
- NOZOMU ETO ET AL: "Immuno-Chromatographic Assay for Diagnosis of Feline Leukemia Virus Infection" CYTOTECHNOLOGY, KLUWER ACADEMIC PUBLISHERS, DO, vol. 43, no. 1-3, 1 November 2003 (2003-11-01), pages 65-72, XP019236806 ISSN: 1573-0778
- SAKATA H ET AL: "Efficiency of donor screening for human parvovirus B19 by the receptor-mediated hemagglutination assay method" VOX SANGUINIS, vol. 77, no. 4, December 1999 (1999-12), pages 197-203, XP009082076 ISSN: 0042-9007
- SLOOTS T ET AL: "Evaluation of four commercial enzyme immunoassays for detection of immunoglobulin M antibodies to human parvovirus B19" EUROPEAN JOURNAL OF CLINICAL MICROBIOLOGY AND INFECTIOUS DISEASES, vol. 15, no. 9, 1996, pages 758-761, XP009082101 ISSN: 0934-9723
- FERGUSON M ET AL: "Report of a collaborative study to calibrate the Second International Standard for parvovirus B19 antibody" BIOLOGICALS, ACADEMIC PRESS LTD., LONDON, GB, vol. 32, no. 4, December 2004 (2004-12), pages 207-212, XP004657308 ISSN: 1045-1056

## Description

### Technical Field

This invention relates to the detection of human parvovirus infection in humans and, in particular, to the detection of acute infection.

By human parvovirus herein is also meant human erythrovirus and the two terms are synonymous.

### Background Art

Human parvovirus is a non-enveloped, single-stranded DNA virus which infects erythroid progenitor cells of the bone marrow and blood. The icosahedral viral capsid is comprised of two structural proteins, VP 1 (5%) and VP2 (95%) (Ozawa, K., et al., (1987) J. Virol 61:2395-2406).

Currently there are three known genotypes of human parvorovirus, Genotype 1 (prototype, parvovirus B 19), genotype 2 (A6 and Lali) and genotype 3 (V9). Although the genotypes vary from the prototype at the genomic level by ∼10% they are known to be immunologically indistinct with similar functional and immunological characteristics (Parsyan, A. et al (2006) Journal of Clinical Microbiology 44 1367-75), and (Hokynar, K. et al (2006) XIth Parvovirus Workshop, Switzerland).

Infection is frequently acquired during childhood where it can cause a mild disease known as *Erythema Infectiosum* (Fifth disease) and is usually self-limiting. By adulthood seroprevalence reaches about 70% (Brown, K.E., (1997) In Anderson, L.J., Young, N.S., editors. Human parvovirus B19. Basel: Kerger, p 42-60) and infection in an immunocompetent individual can result in aplastic crisis and arthropathy (Kerr, S., et al (1999) Journal of Medical Virology 57 179-185). In the immunocompromised host and those with underlying blood disorders, human parvovirus is a significant pathogen and can cause serious complications such as chronic anemia, pure red cell aplasia and thrombocytopenia. Transplacental fetal infection during pregnancy can result in fetal death, hydrops foetalis or congenital anemia (Anand, A., *et al.,* (1987) 316 183-186). Current treatment options include blood transfusion and intravenous immunoglobulin. These treatments are also a source of iatrogenic parvovirus transmission.

During the first three days of infection, which may be asymptomatic, viremia in the peripheral blood often rises to greater than 10¹² genome equivalents per ml (geq/ml). Once antibodies are produced this number rapidly diminishes, however DNA can remain detectable for several years post-infection (Cassinotti, P. and Siegl, G., (2000) Eur J Clin Microbiol Infect Dis 19, 886-7). Acutely infected individuals can unintentionally donate viremic blood that could have serious post-transfusion consequences for the recipient or be transmissible through extracted blood products (Hayakawa, F.; et al., (2000) British Journal of Hematology 118(4) 1187-9). Consequently, human parvovirus contamination of blood products is of maj or public health significance, primarily due to the high resilience of human parvovirus to many of the treatments used in plasma processing, such as solvent-detergent treatment, lyophilisation and high temperatures. Thus, there is a need for a rapid, sensitive and inexpensive screening method for parvovirus detection in individual plasma units or pools from which blood products are extracted, (Aubin, J.T., et al., (2000) Vox Sanguinis 78 7-12).

To date, neither the minimum infectious level of human parvovirus nor the protective level of parvovirus IgG has been defined or elucidated. Both parameters are important as the interplay between the level of neutralising antibodies in the donor and recipient and the viral level determines if a blood product is infectious. Previously, 40 separate pools of blood derived from 20,000 individual blood units were screened by PCR (McOmish, F. et al., (1993) J. Clin Microbiol 31(2): 323-328) and revealed 5 donors who were positive for B 19 DNA (2 x 10⁴ to 5 x 10¹⁰ copies/ml). These donors subsequently seroconverted 3-6 months post-donation (McOmish, F. *et al,* (1993) *supra*)*.* It has been estimated that human parvovirus is present in 1:625 to 1:16,000 transfusions (Thomas, I. et al., (2003) Vox Sang 84: 300-7). Furthermore, a higher seroprevalence has been found among haemophiliacs than in the general population, most probably because of the presence of human parvovirus in Factor VIII purified from pooled plasma (Laub, R. and Strengers, P. (2002) Pathol Biol (Paris) 50(5):339-48).

It is important to confirm the diagnosis of acute parvovirus infection in a public health setting which might involve pregnant women and in which an outbreak could lead to serious medical consequences. As many human parvovirus infections in adults are asymptomatic, it is also important to screen donors as high donor viremia can contaminate huge plasma pools and transmit the virus. Human parvovirus contamination of human plasma or derived blood products is recognised as a significant threat to human health.

Currently human parvovirus viral capsid protein production is detected by immunofluorescence (IF) staining and hemaglutination (HA) assays and viral DNA production by PCR, dot blot hybridization and quantitative PCR. The detection of RNA transcripts by RT-PCR is used as an indirect marker for infection. Previously, an antigen detection EIA was used to detect human parvovirus in acute phase serum from patients with aplastic crisis. Human parvovirus was detected in 6/16 sera specimens with high titres of virus, as determined by electron microscopy and DNA hybridisation, and had no detectable human parvovirus antibody. Human parvovirus was not detected in serum specimens with medium or low titres of human parvovirus DNA (n =10) which had seroconverted (Anderson, MJ., et al., (1986) J infect Disease 152:257-265). The human parvovirus DNA was not quantified and so the sensitivity of the assay was not established but it was reported to be less than the human parvovirus DNA hybridisation assay. It was thought that human parvovirus immune complexes caused the decrease in sensitivity.

Using a combination of VP1 and VP2 monoclonal antibodies, a dot blot assay was developed to detect human parvovirus capsid in human sera. The assay was compared with a dot blot hybridisation assay and nested PCR assay and was subsequently deemed to be comparable, or of slightly higher sensitivity to the hybridisation assay, but less than PCR (Gentilomi, G. et al., (1997) J Clin Microbiol. 35:1575-8).

Although PCR-based assays have a much superior sensitivity, such assays do have disadvantages not shared with EIA. First of all, the genetic polymorphism of parvovirus B 19 and the two variants mean that the choice of primers is critical (Aubin, J.T., et al., (2000), Vox Sanguinis 78 7-12; Nguyen, Q.T., et al., (2002) Virology Sep 30 301(2) 2374-80). Secondly, the significance of DNA in plasma post-viremia is unclear. Finally there is always the potential for DNA cross contamination which may result in false-positive results.

### Disclosure of Invention

The invention provides a method for detecting human parvovirus antigen in a sample, which method comprises contacting a buffer having a pH in the range 3.0 to 4.0 with the sample, followed by measurement of the antigen.

The method according to the invention results in a dramatic improvement in the signal generated in human parvovirus antigen detection, leading to greater assay sensitivity.

The method according to the invention has been found to be effective in detecting human parvovirus antigen in the presence and absence of immune complexes.

Preferably, the buffer has a pH in the range 3.4 - 3.6.

Further, preferably, the buffer is a citrate buffer.

Most preferably, the buffer is a citrate/trisodium citrate buffer.

Preferably, the buffer is added to the sample in ratio of 4:1.

The sample is preferably selected from whole blood, plasma, serum or cord blood.

Alternatively, the sample can be selected from amniotic fluid, bone marrow or synovial fluid.

In one embodiment, the sample is neutralised following the addition of the buffer.

Preferably, the measurement of antigen is carried out by immunoassay, enzyme immunoassay, radio immunoassay or immunofluorescent assay.

Most preferably, the antigen is measured by a capture enzyme immunoassay.

The invention provides a simple and easy-to-use human parvovirus capture assay for the direct detection of human parvovirus.

According to one embodiment, the method has a sensitivity of 10⁸-10⁹ human parvovirus DNA International units *per* ml as measured by PCR and calibrated against the WHO International standard for human parvovirus DNA.

The method according to the invention can be used in the screening of blood donors for human parvovirus.

### Brief Description of the Drawings

Fig. 1 is a comparison of low pH and ordinary sample diluent in detecting two different batches of recombinant VP2 (rVP2) (OD 450/630nm *versus* rVP2 protein concentration ng/ml) as described in Example 1;
Fig. 2 is a comparison of low pH and ordinary sample diluent in detecting native human parvovirus at different viral concentrations as determined by qPCR (OD 450/630nm *versus* human parvovirus DNA (IU/ml)) as described in Example 1;
Fig. 3 is a comparison of low pH and ordinary sample diluent in detecting native human parvovirus in samples with and without human parvovirus IgM antibodies (OD 450/630nm *versus* human parvovirus DNA (IU/ml)) as described in Example 1;
Fig. 4 is a graph of Virus Capture Index Value *versus* human parvovirus DNA (IU/ml) as described in Example 1;
Fig. 5 is a graph of IgM Index Value *versus* human parvovirus DNA (IU/ml) as described in Example 1;
Fig. 6 is a graph of O.D. 450/630nm *versus* VP2 protein concentration (ng/ml) for two batches of recombinant VP2 tested in a virus capture assay according to the invention as described in Example 2;
Fig. 7 is a comparison between the detection of parvovirus genotype 1 and genotype 3 VP2 in a virus capture assay according to the invention as described in Example 3; and
Fig. 8 is a comparison between parvovirus genotype 1 and genotype 2 reactivity in a virus capture assay according to the invention as described in Example 3.

### Modes for Carrying Out the Invention

The invention will be further illustrated by the following Examples.

### Example 1

### Screening of the level of human parvovirus viremia in a blood donor population.

Dutch blood donors were screened over a 17 month period by the Dutch Blood Bank, which let to the identification of 70 individuals with levels of human parvovirus DNA greater than 10⁶ IU/ml at the time of donation. Sample specimens from these 70 donations were further tested for human parvovirus in accordance with the invention by a virus antigen-capture EIA and also for human parvovirus IgM and IgG antibodies.

### Human parvovirus antibody production

Recombinant human parvovirus VP2 expressed in baculovirus was purified as previously described (Kerr, S. *et al* (1999) *supra*) and used for rabbit and sheep immunisations. Once a satisfactory titre was obtained, serum was collected and IgG purified by Protein A affinity chromatography. Purified anti-sheep polyclonal IgG was employed as a human parvovirus-capturing antibody coated on to microtitre plate wells. Rabbit anti-VP2 conjugated to horseradish peroxidase (Hermanson G.T. 1996. Heterobifunctional Cross-linkers, In: Bioconjugate Techniques 1st ed. California Academic Press p 236-237) was used to detect captured virus in the antigen-capture EIA.

### Plate coating

Rabbit anti-VP2 IgG was coated onto microtitre plates (Nunc Maxisorp) in 50 mM sodium carbonate pH 9.6 (coating buffer) and incubated at 2-8 °C for 18h. The microwells were then washed twice with 20mM Tris-HCl, pH 7.2 containing 0.15M NaCl and 0.1% (v/v) Tween (Tween is a Trade Mark)-20 (IBST) and blocked for 1h at 37°C in coating buffer containing 1% (w/v) bovine serum albumin (Sigma). After a subsequent wash cycle with TBST, the plates were dried at 37°C and stored until required.

### Parvovirus capture assay procedure

Test plasma and control specimens were diluted 1 in 5 in a low pH diluent (100 mM citrate/tri-sodium citrate buffer, pH 3.6 containing 0.1% (v/v) Triton X (Triton X is a Trade Mark)-100, 0.4% (v/v) Tween-20 and 10mM EDTA) prior to addition to anti-VP2 IgG coated microwells (100µl/well).

Diluted specimens were incubated for 1h at 37°C. After four washes with TBST to remove unbound material, the rabbit anti-VP2 human parvovirus IgG-HRP conjugate (100 µl/well) was added to the wells and incubated for 30min at 37°C. The wells were washed (4x TBST) and tetramethylbenzidine (TMB) substrate ((100µl/well; BioFX) added to the wells and incubated at 37°C for 30 min. The reaction was then terminated using 1 N sulphuric acid (100µl/well) and the absorbance measured at 450/630nm. The presence of human parvovirus in a sample was determined by the ratio of the specimen absorbance divided by that of the assay cut-off calibrator absorbance. Specimens yielding indices greater than 1.1 were classed as being positive and those with indices less than 1.1 were deemed negative.

### Assay optimisation

The optimal plate-coating concentration and conjugate dilution were established by testing human parvovirus viremic plasma at the lowest detectable level relative to non-viremic samples. Decimal dilutions of baculovirus-expressed VP2 capsids in TBST from 1000ng/ml to 1pg/ml were also tested on the optimised EIA to determine the limit of detection in terms of protein concentration. The mean assay absorbance of a panel of 201 normal human plasma plus three standard deviations was taken as the cut-off value.

### Antibody Testing

The human parvovirus IgM and IgG status of each sample was determined by commercial EIA (Biotrin International Limited, Dublin, Ireland).

### Samples

Of the total samples taken mini-pools were used to screen the donor population. These pools were made up from 1.4 x 10⁶ donors and reactive pools testing greater than >10⁶ IU/ml were then resolved using an in-house algorithm to determine the individual donor source of a reactive pool. During the screening period 70 samples tested positive for human parvovirus DNA. These samples were then screened for human parvovirus virus particles (virus-capture EIA), human parvovirus IgM and human parvovirus IgG in accordance with the method of the invention.

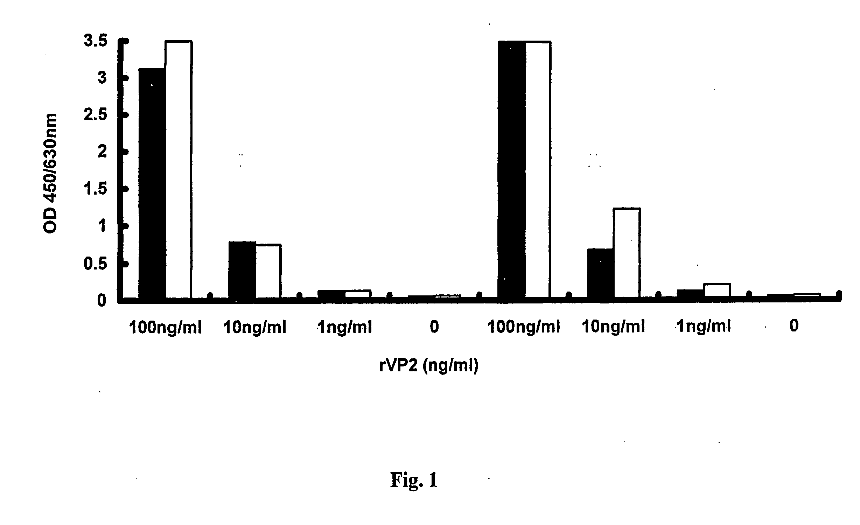

Fig. 1 shows a comparison of low pH (3.6) (filled boxes) and ordinary sample diluent (clear boxes) in detecting rVP2. It will be noted that no significant enhancement by low pH was observed in the detection of recombinant parvovirus VP2 antigen.

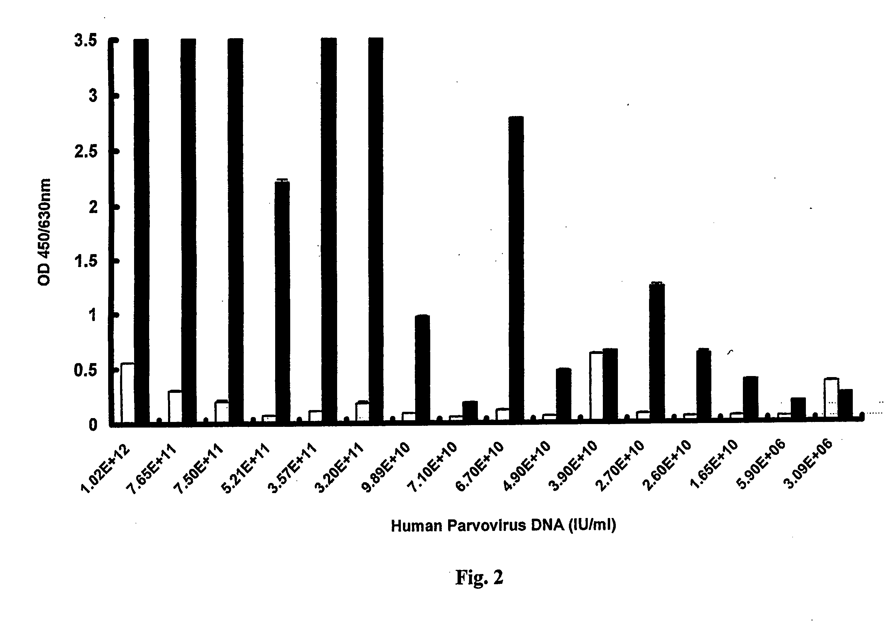

However, when viremic plasma was tested in the same assay format many of the viremic samples were only detected with the low PH buffer as shown in Fig. 2.

Fig. 2 is a comparison of sample diluent buffers used in the detection of human parvovirus viral capsids. Samples were diluted in either 20mM Tris-HCl, pH 7.2 containing 0.15M NaCl and 0.1% (v/v) Tween-20 (TBST) (clear boxes) or 100 mM citrate/tri-sodium citrate buffer, pH 3.6 containing 0.1% (v/v) Triton X-100, 0.4% (v/v) Tween-20 and 10mM EDTA (filled boxes). Error bars represent the standard deviation from the mean. Dilution of the viremic samples in the low pH citrate diluent (pH 3.6) caused a considerable increase in signal in the majority of samples (0 to 30-fold). One sample (3.9*10¹⁰ lU/ml) did not display a significant signal increase post-treatment, but did remain positive. Non-viremic plasma was still negative when given the same pre-treatment.

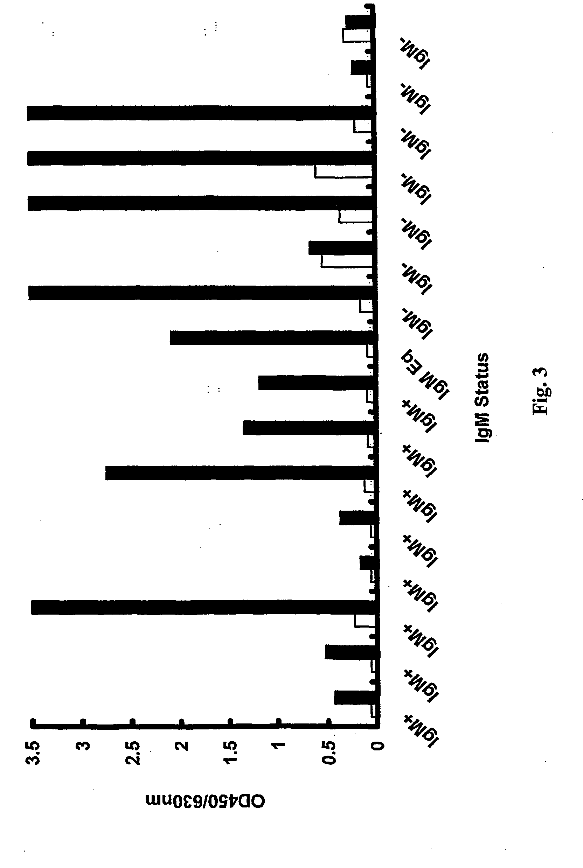

The presence or absence of IgM in the sample did not affect detection of human parvovirus as shown in Fig. 3. The samples tested were a subset of the PCR positive samples.

Fig. 3 shows the effect of low pH in the presence and absence of IgM. The samples were treated with conventional TBST diluent (clear boxes) or low pH (filled boxes) as in the case of Fig. 2. The two samples diluted with TBST with the highest absorbance values were IgM negative.

### Donor sample evaluation

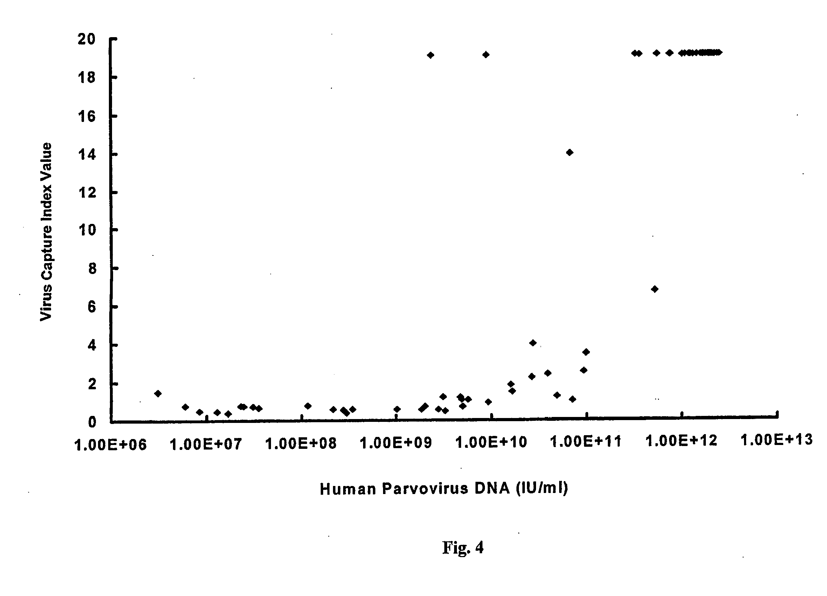

Over the 17 month period, 70 cases of asymptomatic donors with varying levels of human parvovirus DNA greater than 10⁶ IU/ml were identified. Sera was analysed from these viremic donors and revealed, 70% (49/70) tested positive for human parvovirus by EIA (range: 3.2 x 10¹² to 3.1 x 10⁶, mean 1.1 x 10¹², median: 1.2 x 10¹²). The results are shown in Fig. 4.

Fig 4. shows the reactivity of viremic donors on the human parvovirus-capture EIA. An Index Value of greater than 1.1 is positive and less than 1.1 is deemed to be negative.

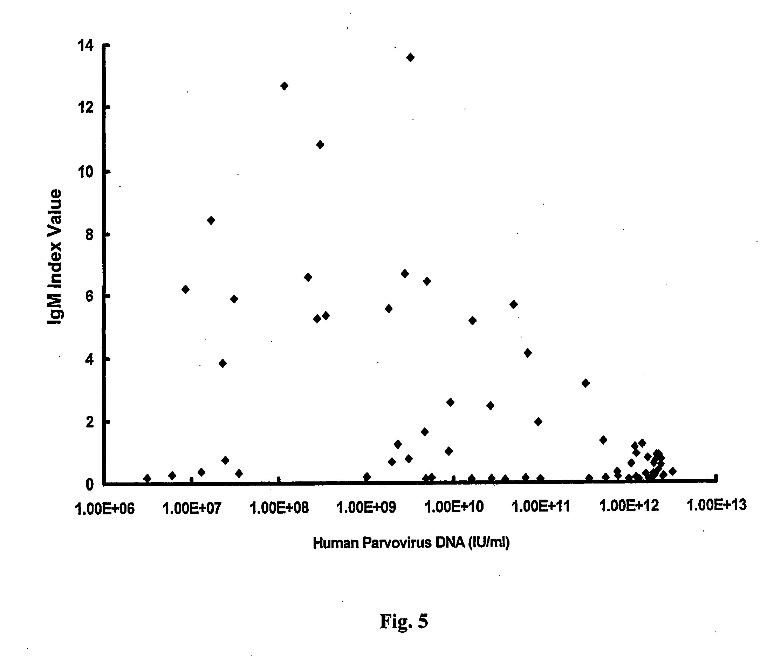

Of the donor samples, 27 (38.6%) tested positive or borderline positive (two samples were equivocal) for human parvovirus IgM by EIA as shown in Fig. 5. None of the 70 veremic samples were positive for human parvovirus IgG antibodies.

Fig 5. shows the IgM reactivity of viremic donors. An Index Value of greater than 1.1 is positive and less than 0.9 is negative. Any samples within this range are deemed equivocal. The samples that were borderline for IgM reacted strongly on the antigen EIA (> 19 Index Value). The overlap between the two groups was considerable, 17% tested positive for both IgM and human parvovirus. The number of donors that that were positive in one or both assays was 91 %.

There was no sensitivity cut-off determined for the assay as there was no direct correlation between the DNA level determined by PCR and the Index Value obtained by the virus capture assay. The assay sensitivity was estimated at approximately 10⁸ genome equivalents per ml, using dilutions of highly viremic samples. Virus capture index values of viremic samples displayed a strong positive correlation to the PCR determined level of parvovirus viremia (r = 0.81). The detection limit of the assay was not directly related to the DNA level determined by quantitative PCR and so it was difficult to define the assay cut-off One sample, quantified as 3.1 x 10⁶ IU/ml was detected on the virus capture assay but a number of specimens in the 10⁷ - 10⁹ IU/ml range were not picked up. The level of human parvovirus DNA, as determined by PCR, may not seem to correspond to infectious capsids.

Kenji Furuya (Japan Red Cross Plasma Fractionation Centre Presented data at SOGAT May 2004 'Analysis of human parvovirus B19 components and strategies of non-enveloped virus removal from Factor VIII concentrates') used salt and pH gradient chromatography to fractionate human parvovirus viremic serum and subsequently identified three distinct fractions; intact capsid, disrupted capsid/DNA complex, and a large proportion of free DNA. This may explain why the viremia quantified by PCR and the virus capture absorbance values described herein do not directly correlate. The detection limit of the human parvovirus capture assay was determined by diluting purified recombinant VP2 and was calculated to be in the 10-100pg/ml range. Recently Lowin, T. et al., (2005) J. Vet Med B Infect Dis Vet Public Health. 52(7-8):348-52 used a similar format to compare VP2 expressed in recombinant yeast to VP2 from a baculovirus expression vector. The detectable level of recombinant VP2 appeared to be in the range of 120ng/ml. Unusually, the use of low pH buffer in accordance with the invention does not enhance recombinant VP2 detection in the virus-capture assay. Assay specificity was 100%, as none of the 201 human plasma specimens from immune individuals tested were positive.

### Example 2

### Determination of assay sensitivity

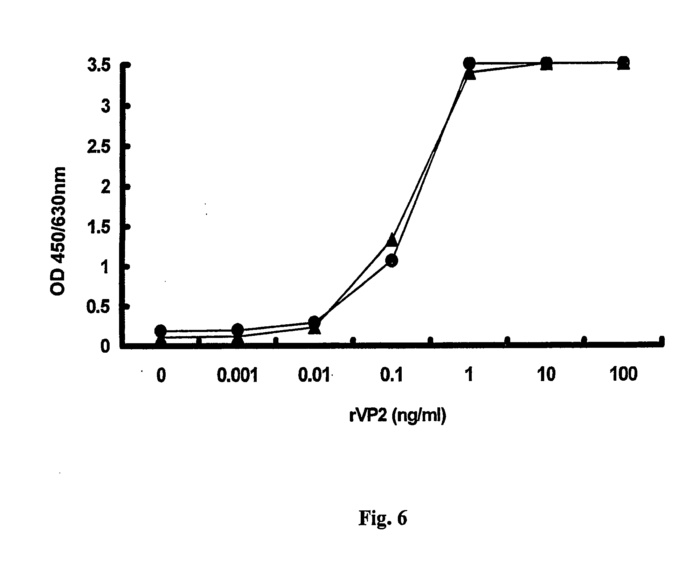

Batches of recombinant VP2 were prepared as follows:
Infected Sf9 cells were harvested 3 days post-infection and lysed by sonication in PBS. Cell debris was removed by centrifuging at 3000 g for 10 minutes and the resultant supernatant was precipitated with PEG/NaCl overnight. Pellets containing VP2 were then resuspended in PBS and quantified. To estimate the assay sensitivity two separate batches of VP2 were diluted to 1 µg/ml and then decimally diluted to 1pg/ml to determine the assay cut-off. The virus-capture EIA in accordance with the invention as described in Example 1 was able to detect recombinant VP2 to a concentration of 10 pg/ml. The results are shown in Fig. 6, wherein the cut off of the assay is clearly depicted.

### EXAMPLE 3

The three parvovirus variants were analysed on the virus capture assay according to Example 1. Genotype 3 VP2 was obtained from Jean Pierre Allain, Cambridge Blood Centre, Cambridge, Long Road, CB2 2PT, United Kingdom (reference Parsyan, A., et al (2006) *supra*)*.* The genotype 2 (A6) sample was received as a gift (Baxter), which had previously been sequenced and quantified (titre of 2.8 x 10¹¹ IU/ml). Genotype 1 was obtained from the Dutch Blood Bank.

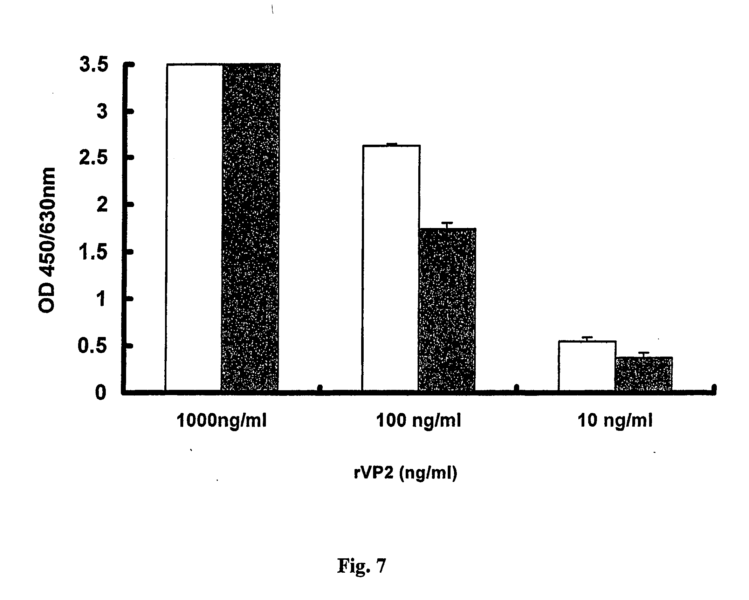

Genotype 1 VP2 capsid protein was found to exhibit similar reactivity to genotype 3 on the virus capture EIA. The results are shown in Fig. 7.

As indicated above, Fig. 7 is a comparison of genotype 1 (clear boxes) and genotype 3 (filled boxes) recombinant VP2. Samples were diluted to 1000, 100 and 10ng/ml of protein in the low pH (3.6) buffer. Error bars represent the standard deviation from the mean. Both genotypes were shown to have very similar reactivity on the virus capture assay. This proves that the virus capture assay does not discriminate between genotype 1 and 3 capsid protein and therefore is capable of detecting acute infection caused by either of these variants.

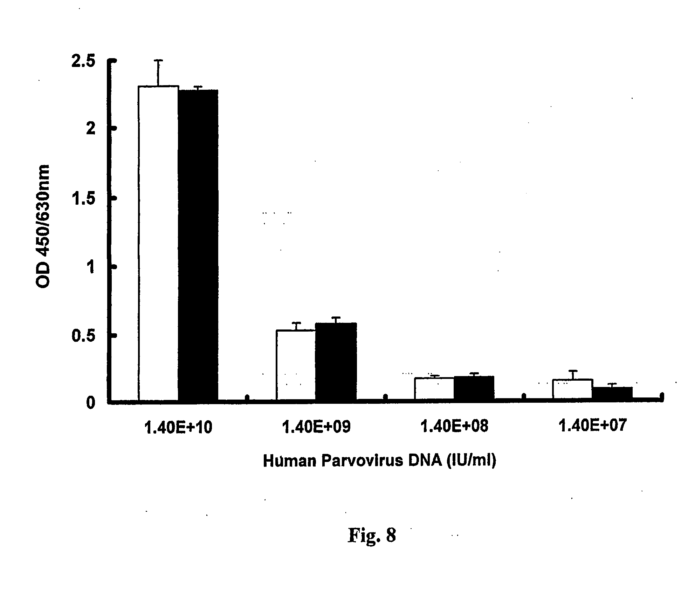

Genotype 1 and genotype 2 viremic samples were also found to exhibit similar reactivity on the virus capture EIA. The results are shown in Fig. 8.

As indicated above, Fig. 8 shows the reactivity of genotype 1 (clear boxes) and genotype 2 (filled boxes) viremic samples on the virus capture EIA. Both the genotype 1 and genotype 2 viremic sample were decimally diluted with a parvovirus negative sample and subsequently compared on the virus capture assay. Error bars represent the standard deviation from the mean. Both genotypes 1 and 2 were shown to have very similar reactivity on the virus capture assay. This proves that the virus capture assay does not discriminate between genotype 1 and 2 capsid detection and therefore is capable of detecting acute infection caused by either of these variants.

As indicated in Example 1, human parvovirus detection was greatly enhanced by acidification of the sample. This would appear paradoxical as low pH buffers are often used to dissociate antigen-antibody complexes, for example in affinity chromatography. It would be expected that acidification would hinder rather than facilitate the capture of the virus by the coating antibody. Although not wishing to be bound by any theoretical explanation of the invention, the low pH conditions may act by breaking up the viral capsid into its structural sub-units, thus making it more accessible to the capture antibody. It was previously thought that human parvovirus was highly resistant to physicochemical treatments, such as acidification (pH 3.5) and also heat treatment (one hour at 56°C) (Siegl, G. (1976) Virology Monographs 15:1-109. More recently the susceptibility of human parvovirus virus to low pH treatment was examined and both infectivity and capsid integrity were monitored post-treatment (Boschetti, N., et al (2004) Transfusion 44:1079-86). Endonucleases were added after the low pH treatment to cleave free viral DNA and the encapsidated viral DNA was quantified. Human parvovirus was inactivated greater than 5 log after 2 hours at pH 4 and infectivity had also decreased. In addition, it is possible that acidification causes the dissociation of any immune complexes present in test samples which may hinder virus capture by coating antibodies. The viral capture was not significantly inhibited by the presence of IgM nor IgG, even at high levels, in low pH conditions as shown in Fig. 3. When the virus capture assay was performed in buffers at physiological pH, the samples that gave the highest absorbance values had no human parvovirus IgM, implying immune complexes hinder detection. A Hemaglutination (HA) assay, which exploits the binding of a human parvovirus receptor to red blood cells (RBCs) *via* the P antigen, is a common way of detecting human parvoviral particles. Immunocomplexes caused by the presence of parvovirus IgG or IgM antibodies are a major problem with this system as they inhibit virus-RBC binding (Sakata, H., et al., (1999) Vox Sanguinis 77:197-203).

Therefore the HA assay sensitivity is greatly affected by specimens which have seroconverted.

The sensitivity of the virus-capture EIA was estimated from the specimen DNA level as determined by quantitative PCR analysis. The sensitivity of the assay as described in Example 1 is between 10⁸ and 10⁹ genome equivalents per ml. There was no direct correlation between PCR determined DNA level and reactivity on the virus-capture EIA. Virus capsids were only detected when samples were pre-treated with the low pH buffer. The assay was able to detect virus in the presence of IgM antibodies and no false-positive results were obtained when normal human plasmas were tested. Combining the results of the virus capture assay with those of the human parvovirus IgM assay, 91 % of acute human parvovirus infections were detected. Although existing quantitative PCR tests for the virus are more sensitive, using an EIA has a number of advantages. PCR detects DNA which may persist at low levels for 6-40 months post-infection and therefore may not be suitable for plasma screening due to detection of viremic, although non-infectious, plasma units for years after infection. Thus, the virus-capture EIA in accordance with the invention may be a better indicator of recent infection.

When the results of the virus capture are combined with the IgM results, 91 % of samples would be diagnosed as acute infection. Experimental infection has shown that human parvovirus infection has two main phases. The first occurs 5-7 days post-inoculation and is characterised by a high viremia (∼10¹¹ copies/ml serum) and few symptoms. The second phase occurs a few days after IgM antibodies first become detectable, during week 2 post-inoculation. Symptoms in phase two include rash and arthralgia (Anderson, MJ., et al., .(1986) J Infect Disease 152:257-265). The development of the IgM response causes a subsequent decrease in viral titre. The IgM response begins to decline about 1 month post-infection and can last for 4-6 months. The 70 viremic specimens described herein showed a typical viremia and IgM seroconversion pattern.

## Claims

1. A method for detecting human parvovirus antigen in a sample, which method comprises contacting a buffer having a pH in the range 3.0 to 4.0 with the sample, followed by measurement of the antigen.

2. A method according to Claim 1, wherein the buffer has a pH in the range 3.4 - 3.6.

3. A method according to Claim 1 or 2, wherein the buffer is a citrate buffer.

4. A method according to Claim 3, wherein the buffer is a citrate/trisodium citrate buffer.

5. A method according to any preceding claim, wherein the buffer is added to the sample in ratio of 4:1.

6. A method according to any preceding claim, wherein the sample is selected from whole blood, plasma, serum or cord blood.

7. A method according to any one of Claims 1-5, wherein the sample is selected from amniotic fluid, bone marrow or synovial fluid.

8. A method according to any preceding claim, wherein the sample is neutralised following the addition of the buffer.

9. A method according to any preceding claim, wherein the measurement of antigen is carried out by immunoassay, enzyme immunoassay, radio immunoassay or immunofluorescent assay.

10. A method according to Claim 9, wherein the antigen is measured by a capture enzyme immunoassay.

11. A method according to any preceding claim, which has a sensitivity of 10⁸ -10⁹ human parvovirus DNA International units *per* ml as measured by PCR and calibrated against the WHO International standard for human parvovirus DNA.

12. Use of a method according to any one of Claims 1-11 for the screening of blood donors for human parvovirus.

## Patentansprüche

1. Verfahren zum Nachweis von menschlichem Parvovirus-Antigen in einer Probe, wobei das Verfahren das in Kontakt bringen eines Puffers mit einem pH im Bereich von 3,0 bis 4,0 mit der Probe umfasst, gefolgt von der Messung des Antigens.

2. Verfahren nach Anspruch 1, wobei der Puffer einen pH im Bereich von 3,4 bis 3,6 aufweist.

3. Verfahren nach Anspruch 1 oder 2, wobei der Puffer ein Zitrat-Puffer ist.

4. Verfahren nach Anspruch 3, wobei der Puffer ein Zitrat/Trinatrium-Zitrat-Puffer ist.

5. Verfahren nach einem der vorhergehenden Ansprüche, wobei der Puffer zu der Probe in einem Verhältnis von 4:1 zugegeben wird.

6. Verfahren nach einem der vorhergehenden Ansprüche, wobei die Probe ausgewählt ist unter Vollblut, Plasma, Serum oder Nabelschnurblut.

7. Verfahren nach einem der Ansprüche 1 bis 5, wobei die Probe ausgewählt ist unter Fruchtwasser, Knochenmark oder Gelenkflüssigkeit.

8. Verfahren nach einem der vorhergehenden Ansprüche, wobei anschließend an die Zugabe des Puffers die Probe neutralisiert wird.

9. Verfahren nach einem der vorhergehenden Ansprüche, wobei die Messung des Antigens durch Immunoassay, Enzymimmunoassay, Radioimmunoassay oder Immunofluoreszenzassay durchgeführt wird.

10. Verfahren nach Anspruch 9, wobei das Antigen durch einen Capture-Enzymimmunoassay gemessen wird.

11. Verfahren nach einem der vorhergehenden Ansprüche, das eine Sensitivität von 10⁸ - 10⁹ internationale Einheiten der menschlichen Parvovirus-DNA pro ml, gemessen durch PCR und kalibriert gegen den internationalen WHO-Standard für menschliche Parovirus-DNA aufweist.

12. Verwendung eines Verfahrens nach einem der Ansprüche 1 bis 11 zum Screenen von Blutspendern auf menschlichen Parvovirus.

## Revendications

1. Procédé de détection d'antigène parvovirus humain dans un échantillon, lequel procédé comprend la mise en contact d'un tampon ayant un pH qui se situe dans la plage de 3,0 à 4,0 avec l'échantillon, suivi par la mesure de l'antigène.

2. Procédé selon la revendication 1, dans lequel le tampon a un pH se situant dans la plage de 3,4 à 3,6.

3. Procédé selon la revendication 1 ou 2, dans lequel le tampon est un tampon citrate.

4. Procédé selon la revendication 3, dans lequel le tampon est un tampon citrate / citrate trisodique.

5. Procédé selon l'une quelconque des revendications précédentes, dans lequel le tampon est ajouté à l'échantillon dans un rapport de 4:1.

6. Procédé selon l'une quelconque des revendications précédentes, dans lequel l'échantillon est sélectionné à partir de sang complet, de plasma, de sérum ou de sang de cordon.

7. Procédé selon l'une quelconque des revendications 1 à 5, dans lequel l'échantillon est sélectionné à partir de fluide amniotique, de moelle osseuse ou de fluide synovial.

8. Procédé selon l'une quelconque des revendications précédentes, dans lequel l'échantillon est neutralisé après l'addition du tampon.

9. Procédé selon l'une quelconque des revendications précédentes, dans lequel la mesure de l'antigène est réalisée par immunoessai, immunoessai enzymatique, radio-immunoessai ou essai par immunofluorescence.

10. Procédé selon la revendication 9, dans lequel l'antigène est mesuré par immunoessai enzymatique de capture.

11. Procédé selon l'une quelconque des revendications précédentes, lequel présente une sensibilité de 10⁸ à 10⁹ unités Internationales d'ADN de parvovirus humain par ml, telle que mesurée par PCR et calibrée en fonction de la norme Internationale WHO pour l'ADN de parvovirus humain.

12. Utilisation d'un procédé selon l'une quelconque des revendications 1 à 11, pour le criblage de donneurs de sang pour le parvovirus humain.
